(19) Europäisches Patentamt
European Patent Office
Office européen des brevets

(11) **EP 2 710 154 B1**

(12) **EUROPEAN PATENT SPECIFICATION**

(45) Date of publication and mention
of the grant of the patent:
**13.09.2017   Bulletin 2017/37**

(21) Application number: **12786747.1**

(22) Date of filing: **14.05.2012**

(51) Int Cl.:
***C12Q 1/68*** *(2006.01)*

(86) International application number:
**PCT/US2012/037712**

(87) International publication number:
**WO 2012/158587 (22.11.2012 Gazette 2012/47)**

(54) **WEIGHT MANAGEMENT GENETIC TEST SYSTEMS AND METHODS**

GENETISCHE TESTSYSTEME UND VERFAHREN ZUR GEWICHTSKONTROLLE

SYSTÈMES ET PROCÉDÉS DE TEST GÉNÉTIQUE POUR LA GESTION DU POIDS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.05.2011   US 201161487543 P
19.05.2011   US 201161487960 P
16.11.2011   US 201161560620 P**

(43) Date of publication of application:
**26.03.2014   Bulletin 2014/13**

(73) Proprietor: **Genovive LLC
Harahan, LA 70123 (US)**

(72) Inventors:
• **CASTELLON, Victor
  Harahan, LA 70123 (US)**
• **CLINKSCALES, Paul
  Harahan, LA 70123 (US)**
• **DAVIS, James
  Harahan, LA 70123 (US)**
• **GILL, Rosalynn
  Harahan, LA 70123 (US)**
• **NG, San, San
  Harahan, LA 70123 (US)**
• **SWANGO, Beverly
  Harahan, LA 70123 (US)**
• **DEBUSK, Ruth
  Harahan, LA 70123 (US)**

(74) Representative: **Henderson, Helen Lee
Withers & Rogers LLP
4 More London Riverside
London SE1 2AU (GB)**

(56) References cited:
WO-A2-2010/048378    US-A1- 2006 078 497
US-A1- 2008 050 358    US-A1- 2008 171 335
US-A1- 2009 075 254    US-A1- 2009 299 645
US-A1- 2010 098 809    US-A1- 2010 098 809
US-A1- 2010 136 561    US-A1- 2010 249 065

• QI ET AL.: 'Interleukin-6 Genetic Variability and Adiposity:Associations in Two Prospective Cohorts and Systematic Review in 26,944 Individuals' J CLIN ENDOCRINOL METAB vol. 92, no. 9, 2007, pages 3618 - 3625, XP055135683
• GRARUP ET AL.: 'The -250G>A Promoter Variant in Hepatic Lipase Associates with Elevated Fasting Serum High-Density Lipoprotein Cholesterol Modulated by Interaction with Physical Activity in a Study of 16,156 Danish Subjects' J ENDOCRIN RES vol. 93, 2008, pages 2294 - 2299, XP055136295
• TOREKOV ET AL.: 'Variants in the 5' region of the neuropeptide Y receptor Y2 gene (NPY2R) are associated with obesity in 5,971 white subjects' DIABETOLOGIA vol. 49, 2006, pages 2653 - 2658, XP019440401
• SMALL, KS ET AL.: 'Identification of an imprinted master trans regulator at the KLF14 locus related to multiple metabolic phenotypes' NATURE GENETICS vol. 43, 15 May 2011, pages 561 - 564, XP055135684
• RUNG ET AL.: 'Genetic variant near IRS1 is associated with type 2 diabetes, insulin resistance and hyperinsulinemia' NAT GENET. vol. 41, 2009, pages 1110 - 1115, XP055135686

Remarks:
The file contains technical information submitted after the application was filed and not included in this specification

Remarks:
The file contains technical information submitted after the application was filed and not included in this specification

**Description**

This disclosure relates generally to systems and methods for providing individuals with diet and exercise programs and, more specifically, relates to utilizing DNA testing for specific genes and gene variants for providing individualized diet and exercise programs based on each individual's unique genetic makeup.

**BACKGROUND**

Obesity is a major public health concern for both the health and the economic burdens that it can cause. Overweight and obesity are measured through the use of the Body Mass Index ("BMI"), which is calculated from weight and height. In the United States, a BMI of 25 but less than 30 is considered to be overweight, while a BMI of 30 or greater is considered to be obese. The general term "overweight," as used in this disclosure, pertains to a BMI of 25 or greater.

By these BMI criteria, approximately 72.5 million Americans are obese, which is approximately one-third of the total U.S. population, and approximately 17% of children aged 2-19 are obese. In 2010, no state had an obesity rate below 18% and some states had an obesity rate exceeding 30%. Worldwide, the World Health Organization ("WHO") reported in 2003 that globally, 1 billion adults were overweight and 300 million adults were obese, and it is estimated that by 2015, 2.3 billion adults will be overweight and 700 million will be obese based on BMI values.

Being overweight or obese is strongly associated with the development of chronic diseases such as heart disease; stroke; endometrial, breast, and colon cancers; Type 2 diabetes; osteoporosis; asthma and other respiratory conditions; and osteoarthritis and other musculoskeletal disorders. In addition to the potential for premature death, such chronic conditions may take a significant toll on quality of life and may cause substantial disability and lost productivity. The economic burden of health care costs for weight-related diseases was $78.5 billion in 1998. At the rate observed through 2006, those costs were projected to reach as high as $147 billion in 2008. The cost of care for an obese individual is estimated to be $1,429 higher annually than for a normal weight individual.

Weight management involves balancing energy intake and energy utilization, with excess energy being stored as body fat. Caloric intake beyond the body's needs and/or utilization of calories below the body's needs will lead to a net gain in weight. Excess calories may be converted to fat and stored in the body's fat cells (adipocytes) as triglycerides. Although once thought to be static in number and function from birth, adipocytes can grow in both size and number as they fill with fat and divide to form new cells.

As the number of adipocytes increase, adipose tissue increases, and adipose tissue is now recognized to be more than a collection of fat cells. Macrophages may infiltrate the tissue and secrete hormones and pro-inflammatory cytokines, which may increase an overweight individual's susceptibility to obesity and other chronic disorders, such as insulin resistance, metabolic syndrome, diabetes, heart disease, and other inflammatory conditions.

Healthy weight management requires balancing caloric intake and energy expenditure, typically through physical activity. However, there are many behind-the-scenes factors that affect this seemingly simple equation. The composition of those calories-the amount and type of dietary fat and carbohydrates-is one such factor. Numerous recent studies have shown that the total fat and total carbohydrate calories may be important for weight management, but for many years the focus was only on total calories, with the type of macronutrient thought to be important only because of its calorie density. It was believed that fat intake should be minimized because, ounce for ounce, fat is more than twice as calorie-dense as proteins or carbohydrates.

In time, though, whether the fat eaten was a saturated-, trans-, monounsaturated-, or polyunsaturated-fat became important from a cardiovascular health perspective. More recently, attention has been focused on unsaturated-fats, particularly omega-3 and omega-6 fats, which have beneficial functions in the body. Further, carbohydrates have been found to increase blood triglycerides, which are the main storage form of body fat, and to affect insulin levels, which in turn can affect appetite, body fat levels, and the inflammatory state of insulin resistance. Much of this macronutrient balancing act ultimately affects the pro- or anti-inflammatory state of the tissues, which is yet another factor in healthy weight management.

Clearly, healthy weight management is a complex process and the interconnectedness of the various components increases the basal complexity. As will become apparent below in discussing the specific gene variants selected, changes to the genetic information can affect many of these components in the weight management puzzle.

BMI is commonly used as the measure of whether an individual is at a desirable body composition because of its convenience, but also because it is considered to be a better indicator than scale weight alone because it takes into account weight in relation to height. What is emerging as perhaps more insightful information, however, is the need to measure an individual's amount and location of body fat. It is stored body fat and its influence on adipose tissue secretion of pro-inflammatory cytokines that is thought to be the link between being overweight or obese and having a higher risk for developing the chronic disorders listed above. Therefore, an improved weight management algorithm is desired that is more tailored to an individual's genetic profile and how it governs their responses to macro and micro nutrients.

## SUMMARY OF THE INVENTION

It is desirable to have an individually-tailored assessment of overweight and obesity that utilizes traditional BMI measurements, percentage of body fat, elevated waist circumference or waist-to-hip ratio, and whether the individual has excessive visceral body fat or pro-inflammatory indicators such as increased cytokine levels, insulin resistance, or metabolic syndrome.

A critical component in weight management is the individual's genetic makeup. Ancient human genes have changed little in approximately 40,000 years, yet these genes are required to interact with different environments, vastly different food supplies, more sedentary lifestyles, and exposure to a host of toxic chemicals that are metabolically active and readily stored in body fat. Although each person has the same basic set of genes (genotype) characteristic of the human species, each individual has a slightly different version of that common theme. Each gene is a potential source of genetic variation within the unique person's genetic makeup (genome). In keeping with the universal theme of evolution and survival of the fittest, these genetic variations ("gene variants") may have a positive, negative, or neutral effect on that gene's role in weight management. Thus, an individual's personal genotype can affect how challenging it will be for that individual to maintain a healthy weight in the environment in which they live.

Weight management is a complex process, and genes are involved in multiple aspects of the process through the proteins they may encode. More than 100 genes have been identified as affecting the ability to maintain a healthy weight. However, no single gene has been discovered that alone is responsible for weight gain in a majority of members across multiple populations. Instead, the prevailing consensus is that there are multiple genes whose collective effect leads to increased susceptibility to weight gain and, ultimately, obesity. Further, variations in these genes do not appear to be sufficient in themselves to cause weight gain. Rather, they provide the susceptibility, but require interaction with one or more environmental factors before that susceptibility is triggered and results in weight gain.

These genes and gene variants can be systematically identified based on the current understanding of the underlying metabolic mechanisms. For example, variants may affect weight management outcomes by influencing such processes as the digestion, absorption, and utilization of food, particularly fat-rich food since fat is the most concentrated source of calories in the diet. Variants that affect any number of the metabolic processes in adipose tissue related to fat storage and mobilization should also be potential candidates.

However, not all of these gene variants have been identified and characterized sufficiently to be useful for predicting susceptibility to weight management challenges. Nutrigenetic testing technology now exists that allows for identification of gene variants that increase the susceptibility of weight gain, and this testing technology has been used to develop a test panel for commercial applications in the area of weight management guidance. Utilizing DNA testing for specific genes and gene variants, an algorithm may recommend exercise and diet programs based on an individual's unique makeup of genes and gene variants.

In more detail, the invention is a method of weight management according to claim 1. The method comprises obtaining at least three, preferably 4, 5 or more, of a client's values relating to weight, height, waist circumference, hip circumference, abdominal fat, body mass index, waist-to-hip ratio, gender, and ethnicity, and the like, and detecting the allele of at least 10, preferably 11 or 12 or more, genetic variants in said client, the genetic variants selected from the group consisting of rs1042714, rs1799883, rs1800588, rs1800629, rs1800795, rs1801282, rs2070895, rs4343, rs4994, rs5082, rs1042713, rs9939609, rs2943641 or an allele linked thereto, and based on those results, selecting a suitable diet plan and an exercise plan.

In further embodiments, each of the detected genetic variants is assigned a Carbohydrate Score, Fat Score and Exercise Score based on the allele detected, and optionally also based on the various body measurements, gender, race and the like. The Carbohydrate Scores are summed, as are Fat Scores and Exercise Scores (collectively also known as nutrigenetic values and/or sums), and diet and exercise plans selected according to predetermined thresholds set for same.

In another embodiment, the description relates to business methods used to provide diet and exercise and weight management services to a client. More particularly, a method of providing weight management services, includes collecting a biological sample from a client and obtaining at least three of weight, height, hip circumference, waist circumference, gender, ethnicity, and the like from said client. The presence or absence of at least 10 genetic alleles known to be associated with weight gain is then determined using said biological sample. The various results are inputted into a computer, assigning values thereto and a score computed based on same, and a weight management and exercise plan is outputted based on the scores so obtained. In some embodiments, the method includes providing prepared meals or components thereof to said client that accord with the diet plan, as well as providing detailed exercise plans and/or individual coaching. In preferred embodiments, the scores are categorized according to fat related, carbohydrate related and exercise related and separately summed and diet and exercise plans based on the three component scores.

Yet another embodiment provides a method of providing weight management services, comprising: a) processing in a first processor a biological sample received from a client; b) receiving data representative of at least three of weight, height, hip circumference, waist circumference, gender, and ethnicity from said client; c) through the processing of the biological sample in the first processor, detecting the presence or absence of at least 10 genetic alleles known to be associated with weight gain in said biological sample; d) computing in a second processor a score based upon the processing of the biological sample and the client data received in action b); e) determining in the second processor a weight management and exercise plan based on the score obtained from action d); and f) transmitting the weight management and exercise plan to the client; and g) optionally providing through a third processor for the selection and delivery of prepared meals or components thereof to the client in accordance with the diet plan of action f). Of course, the various processors can be the same or separate, and some information can be transmitted via the internet or cell phones or other such communication methods.

The use of the word "a" or "an" when used in conjunction with the term "comprising" in the claims or the specification means one or more than one, unless the context dictates otherwise.

The term "about" means the stated value plus or minus the margin of error of measurement or plus or minus 10% if no method of measurement is indicated.

The use of the term "or" in the claims is used to mean "and/or" unless explicitly indicated to refer to alternatives only or if the alternatives are mutually exclusive.

The terms "comprise," "have," and "include" (and their variants) are openended linking verbs and allow the addition of other elements when used in a claim. The phrase "consisting of" excludes other elements. The term "consisting essentially of" occupies a middle ground, allowing the inclusion of nonmaterial elements, such as instructions, and the like, that do not materially change the novel features or combination of the invention.

The following abbreviations are used herein:

| SNP | Single nucleotide polymorphism |
| --- | --- |

As used herein "obtaining" client sample, or DNA or sequence or various data includes both direct and indirect methods of obtaining same. Thus, for example, a sample can be collected at a retail center and passed on to the entity that will conduct the relevant DNA testing and such is to be included as within the scope of this term.

As used herein "sequencing" any DNA or RNA includes both direct and indirect methods of obtaining sequencing information. Thus, the sample can be sequenced by a third party and such is to be included as within the scope of this term.

Likewise, "determining" any of various data includes both direct and indirect methods. Thus, an independent entity can do the actual genetic analysis and provide the data obtained thereby to the computer for processing in the algorithms described herein, and such is to be included within the scope of the term "determining."

## BRIEF DESCRIPTION OF THE DRAWINGS

FIGURE 1 illustrates a high level flow diagram of the genetic diet algorithm 100, in accordance with the present disclosure;

FIGURE 2 illustrates a detailed system level diagram 200 of the flow diagram of FIG. 1, in accordance with the present disclosure; and

FIGURE 3 illustrates a detailed flow diagram 300 combining the elements of FIGS. 1 and 2, in accordance with the present disclosure.

## DETAILED DESCRIPTION

Genes and gene variants may be evaluated on their effect on weight management and then an algorithm may be used to develop an individually tailored diet and exercise programs based on an individual's genetic makeup. Thirteen gene variants in eleven genes have currently been identified to have a sufficiently strong effect on weight management. The criteria used to evaluate whether gene variants have a sufficiently strong effect on weight include function, impact, frequency, weight management association, diet and lifestyle implications, and human intervention studies.

In developing the system and method described herein, data for each of the 13 gene variants were gathered from studies available in the scientific literature, and variants were separated into those that affected diet-related weight management and those that affected exercise-related weight management. The relative values may be assigned to each variant based on two contributions: (1) the variant's overall strength in meeting the selection criteria; and (2) the magnitude of its contribution to dietary- or exercise-related effects on weight management. The diet-related variants may be scored in terms of their effect on dietary carbohydrate-related weight management and separately for their effect on dietary fat-related weight management. If there is evidence of a gender-specific effect or an ethnic-specific effect in either the diet- or exercise-related variants, that information may also be factored into the valuation. These values may play an integral part in the disclosed embodiments of the genetic diet algorithm to translate the genetic test results into personalized diet and physical activity programs for an individual.

The selected gene variants primarily influence the absorption of dietary fat, the storage of excess calories as body fat, or the ability to mobilize stored body fat in response to physical activity. Of the 13 gene variants in the panel, 10 may provide diet-related assistance, 5 may provide exercise-related assistance, and 2 may provide insight into both aspects of weight management. The relevant gene variants are listed below in TABLE 1, in relative order of contribution to weight management within their respective diet- or exercise-related categories.

| Table 1: Gene Variants | | | |
|---|---|---|---|
| Gene | Variant | rs# | Association with Weight Management |
| Diet-Related Variants | | | |
| IRS1 | CC allele | rs2943641 | Individuals with the IRS1 rs2943641 CC genotype might obtain more benefits in weight loss and improvement of insulin resistance than those without this genotype by choosing a high-carbohydrate and low-fat diet. |
| FTO | T>A Chr. Pos'n 53820527 | rs9939609 | FTO (fat mass- and obesity-associated gene) has the strongest association with obesity of any gene to date and is the most commonly occurring body fat-related gene variant, with a strong association with weight and BMI. Those with the AA genotype in the FTO intron had a higher BMI than those heterozygous for the A allele and the low-risk T allele. |
| PPARG | Pro12Ala P12A | rs1801282 | PPARG (peroxisome proliferator-activated receptor-gamma) is a key gene encoding a transcription factor that regulates several genes involved in fat and carbohydrate metabolism and in fat cell formation. Ala carriers have greater increase in overall glucose tolerance, glucose effectiveness, acute insulin response to glucose and disposition index and are more responsive to exercise. |
| LIPC | -557 or -514 C>T | rs1800588 | LIPC (hepatic lipase) helps control blood fat levels. Variants with decreased activity of LIPC have higher levels of blood fats and greater potential for fat storage. The SNP is associated with serum lipid and apolipoprotein concentrations, especially in women, in the order CC, CT, and TT. |
| FABP2 | Ala54Thr A54T +1283G>A | rs1799883 | Intestinal absorption of long-chain dietary fatty acids is increased 2-fold in individuals with this FABP2 (fatty acid binding protein 2) SNP, resulting in increased fat oxidation and insulin resistance. |
| TNF | -308G>A | rs1800629 | The A allele of TNF$\alpha$ (tumor necrosis factor alpha) is associated with higher levels of TNFA expression and thus systemic inflammation, which is associated with higher levels of body fat as well as several disease states. |
| ADRB2 | G1n27G1u Q27E +79C>G | rs1042714 | Plays a major role in reducing subcutaneous body fat through its action in mobilizing stored body fat. The G allele of ADRB2 ($\beta$2-adrenergic receptor) is associated with reduced risk of type 2 diabetes, and is associated with being more responsive to diet changes. |
| APOA2 | -265T>C | rs5082 | Individuals with the CC genotype of APOA2 (apolipoprotein A2) tend to consume greater amounts of dietary fat and to gain weight and have a higher BMI. |

(continued)

| Table 1: Gene Variants | | | |
|---|---|---|---|
| Gene | Variant | rs# | Association with Weight Management |
| Diet-Related Variants | | | |
| IL6 | -174G>C | ras1800795 | The C allele decreases IL-6 (interleukin 6) pro-inflammatory cytokine levels. Subjects with G at position -174 of the IL6 gene with same age, sex, BMI, and waist-to-hip ratio showed almost twice plasma triglycerides, very low density lipoprotein (VLDL) triglycerides, higher fasting and postglucose load free fatty acids, slightly lower high density lipoprotein (HDL)-2 cholesterol, and similar cholesterol and LDL cholesterol levels than carriers of the C allele. Obese individuals with BMI greater than or equal to 28 carrying the CC genotype showed a more than 5-fold increased risk of developing NIDDM (type 2 diabetes) compared with the GG or GC genotypes. |
| LIPC | 293G>A or -250 | rs2070895 | Helps control blood fat levels. Individuals with this variant have decreased activity of hepatic lipase, have higher levels of blood fats, and greater potential for fat storage. |
| IRS1 | C>T | rs2943641 | The IRS 1 gene is important in generating energy from dietary carbohydrates. One variant has a negative effect on weight loss when the diet is high in carbohydrates. |
| Exercise-Related Variants | | | |
| FTO | T>A | rs9939609 | Individuals with the A allele have an increased risk of obesity that can be reduced with higher levels of physical activity; low levels of physical activity exacerbate the effect of the risk allele. |
| ACE | 2350 A>G tagging SNP | rs4343 | The G allele of ACE (angiotensin-converting enzyme) leads to increased expression of the ACE gene and increased abdominal adiposity. High intensity physical activity is helpful in weight management. |
| ADRB2 | G1n27G1u Q27E +79C>G | rs1042714 | Women with this variant have trouble losing weight through diet alone; combining diet with an intense level of physical activity is helpful in promoting body fat reduction in these individuals. |
| ADRB2 | Arg16Gly R16G | rs1042713 | Individuals with the variant allele do not readily mobilize body fat in response to physical activity. |
| ADRB3 | Trp64Arg R64W 190T>C | rs4994 | Important in energy metabolism and in loss of abdominal and subcutaneous fat; the ADRB3 (beta3 adrenergic receptor) variant is associated with a lowered response to physical activity with respect to weight loss |

Each of the 13 gene variants in the panel may be given a "gene score" or "nutrigenetic value" which is a weighted score based on the impact of each gene variant on issues related to weight management. The scores are based on a scale of 0-5, with a score of 0 having no impact and a score of 5 having the greatest impact on weight loss.

Individuals that have been genotyped may have one or more scores assigned for each gene in the panel. According to one embodiment, shown in TABLE 2 below, each individual may then receive a total Carbohydrate Score, Fat Score, and an Exercise Score based on their genetic makeup and the presence of the each of the gene variants in the panel. The maximum total scores for the categories are as follows: female carbohydrate score of 10, female fat score of 29, female exercise score of 16, male carbohydrate score of 9, male fat score of 28, and male exercise score of 15.

| Table 2: Scoring | | | | | | | |
|---|---|---|---|---|---|---|---|
| GENE | rs # Variant | Common Name | Allele | Ethnicity/ Gender/ BMI | Carbohydrate C Score | Fat F Score | Exercise E Score |
| ACE | rs4343 | D>I | GG (DD) | | 0 | 0 | 4 |
| | | | GA (ID) | | 0 | 0 | 3 |
| | | | AA (II) | | 0 | 0 | 0 |
| ADRB2 | rs1042714 | Gln27Glu | CC | Male (non Asian) | 2 | 2 | 2 |
| | | C>G | | Male (Asian) | 0 | 0 | 0 |
| | | | | Female | 0 | 0 | 0 |
| | | | CG | Male (non Asian) | 0 | 0 | 0 |
| | | | | Male (Asian) | 2 | 2 | 0 |
| | | | | Female | 3 | 3 | 3 |
| | | | GG | Male (non Asian) | 0 | 0 | 0 |
| | | | | Male (Asian) | 2 | 2 | 0 |
| | | | | Female | 3 | 3 | 3 |
| ADRB2 | rs1042713 | Arg16Gly | AA | | 0 | 0 | 2 |
| | | A>G | AG | | 0 | 0 | 2 |
| | | | GG | | 0 | 0 | 0 |
| ADRB3 | rs4994 | Trp64Arg | TT | | 0 | 0 | 0 |
| | | T>C | TC | | 0 | 0 | 2 |
| | | | CC | | 0 | 0 | 2 |
| APOA2 | rs5082 | -265T>C | TT | | 0 | 0 | 0 |
| | | | TC | | 0 | 0 | 0 |
| | | | CC | | 0 | 2 | 0 |
| FABP2 | rs1799883 | Ala54Thr | GG | | 0 | 0 | 0 |
| | | G>A | GA | | 1 | 3 | 0 |
| | | | AA | | 1 | 3 | 0 |
| FTO | rs9939609 | T>A | TT | | 0 | 0 | 0 |
| | | | TA | | 2 | 5 | 5 |
| | | | AA | | 2 | 5 | 5 |
| IL6 | rs1800795 | -174G>C | GG | | 0 | 2 | 0 |
| | | | GC | | 0 | 2 | 0 |
| | | | CC | | 0 | 0 | 0 |
| LIPC | rs1800588 | -514C>T | CC | | 0 | 0 | 0 |
| | | | CT | | 0 | 2 | 0 |
| | | | TT | | 0 | 2 | 0 |
| LIPC | rs2070895 | -250C>T | GG | | 0 | 0 | 0 |
| | | | GA | | 0 | 1 | 0 |
| | | | AA | | 0 | 1 | 0 |

(continued)

| Table 2: Scoring | | | | | | | |
|---|---|---|---|---|---|---|---|
| GENE | rs # Variant | Common Name | Allele | Ethnicity/ Gender/ BMI | Carbohydrate C Score | Fat F Score | Exercise E Score |
| PPAR G | rs1801282 | Pro12Ala | CC | | 0 | 4 | 0 |
| | | C>G | CG | BMI<30 | 2 | 4 | 0 |
| | | | | BMI>30 | 3 | 4 | 0 |
| | | | GG | BMI<30 | 2 | 0 | 0 |
| | | | | BMI>30 | 3 | 0 | 0 |
| TNF | rs1800629 | -308G>A | GG | | 0 | 0 | 0 |
| | | | GA | | 0 | 2 | 0 |
| | | | AA | | 0 | 2 | 0 |
| IRS1 | rs2943641 | C>T | CC | | 0 | 4 | 0 |
| | | | CT | | 2 | 2 | 0 |
| | | | TT | | 2 | 2 | 0 |

As shown above in TABLE 2, each of the gene variants has various gene scores related to the intake of carbohydrates and fat or physical activity, although different alleles of the same gene may have different gene scores for each category, as described below.

**Gene Score of 5:** The highest gene score of 5 is assigned to gene variants with multiple publications of strong associations with overweight or obese status in large populations with data from observational or intervention studies associated with intake of carbohydrates, fats, or physical activity. Additional requirements for the assignment of the gene score of 5 include: observational population based studies of greater than 5000 participants per study and intervention studies with populations of greater than 100 individuals showing a statistically significant effect on weight related parameters. Conflicting reports in the literature should be rare to nonexistent.

Only one gene, the FTO gene, met the criteria for the gene score of 5 for both the fat category and the exercise category parameters. Both of these parameters showed a strong effect for the A allele of the FTO rs9939609 variant. The A allele is associated with overweight and obese status, but the association is reduced when fat levels in the diet are restricted and physical activity levels are higher. There is some evidence that indicates a role of carbohydrate levels in the diet as well; however, the evidence is more limited for this category for this allele, and is reflected in the gene score of 2 for A allele carriers.

**Gene Score of 4:** The gene score of 4 is assigned to variants with strong associations with overweight or obese status in multiple publications, but the population sizes in the publications tend to be smaller than the study sizes for gene variants assigned a gene score of 5. General requirements for a gene score of 4 include: observational population based studies of 500-1000 participants per study, or, alternatively, historical publications (>5) over a 5-10 year period showing similar trends with populations of approximately 100 individuals per study, and intervention studies with populations of 20-100 individuals showing a statistically significant effect on weight related parameters. Conflicting reports in the literature may exist, but should be relatively uncommon and related to study design or study population characteristics, such as ethnicity, age, or starting BMI, for example. Variants of three genes-the ACE, IRS1 and the PPARG genes-have been given a gene score of 4.

The ACE gene may affect the exercise category parameters. For the ACE gene, the D allele, which actually refers to the absence of an inserted Alu repeat element, has been associated with increased levels of abdominal fat, as well as other factors related to the metabolic syndrome. Individuals with the D allele respond to vigorous, power based exercise activities, hence the score of 4 for the DD genotype. Individuals with the I allele are more responsive to endurance type activities. Individuals with one copy of each allele tend to fall somewhere in the middle, hence the gene score of 3 for exercise for the ID carriers.

The PPARG gene may affect the fat category parameters. The PPARG Pro12Ala has been associated with weight management parameters in numerous studies. The Pro allele has been associated with higher BMI, waist circumference, and other parameters associated with overweight and obesity, and levels of fat in the diet are particularly important for these individuals.

IRS1 mediates the control of various cellular processes by insulin. When phosphorylated by the insulin receptor, it binds specifically to various cellular proteins containing SH2 domains, such as phosphatidylinositol 3-kinase p85 subunit or GRB2. Common genetic variants in the IRS1 gene have been recently associated with insulin resistance and hyperinsulinemia. Individuals with the IRS 1 rs2943641 CC genotype might obtain more benefits in weight loss and improvement of insulin resistance than those without this genotype by choosing a high-carbohydrate and low-fat diet.

**Gene Score of 3:** The gene score of 3 is assigned to variants with consistently reported associations with overweight or obese status in multiple publications, but the population sizes in the publications tend to be smaller than those for gene scores of 4 or 5. General requirements for a gene score of 3 include: observational population based studies of 75-100 participants per study, or, alternatively, historical publications showing similar trends with populations of approximately 50 individuals per study, and intervention studies with populations of less than 50 individuals showing an impact on weight related parameters. Conflicting reports in the literature may exist, but should be in the minority and ideally should be related to study design and/or population composition. Variants of four genes-the ADRB2, PPARG, ACE, and FABP2 genes-have been given a gene score of 3.

The ADRB2 gene may affect the fat, carbohydrate, and exercise category parameters. The ADRB2 Gln27Glu variant has differential effects on weight management reported in the literature dependent on gender and ethnicity. The gene score of 3 is assigned to females carrying the CG and GG genotypes due to repeated results demonstrating a greater impact of dietary factors, specifically carbohydrate and fat levels, as well as physical activity levels on women with these genotypes. Asian males demonstrate a similar, but attenuated effect relative to females, hence the gene score of 2 assigned to dietary factors for Asian males of these genotypes. There are few data on the impact of exercise in the Asian male population, so a score of zero is given for this population due to lack of evidence. In contrast, non-Asian males demonstrate an opposite, attenuated impact relative to females, with CC carriers showing an increased impact of carbohydrate and fat dietary factors, and CC and CG carriers showing an impact of physical activity.

The PPARG gene may affect the carbohydrate category parameters. The weight status of individuals with the Ala allele has an impact on how these individuals respond to carbohydrate levels in the diet; high carbohydrate levels in the diet are more deleterious for individuals with a BMI >30. The relative gene score of 3 or 2 (for individuals with a BMI <30) compared with the gene score of 4 for the PPARG fat component reflects both the levels of evidence currently available and the relative impact of the two dietary factors.

The FABP2 gene may affect the fat category parameters. The FABP2 Ala54Thr variant is associated with increased BMI and body fat, together with impaired fat and carbohydrate metabolism alterations, which lead to alterations in body weight parameters such as elevated plasma lipid levels, increased triglycerides and elevated blood glucose levels. The gene score of 3 for Fat has been assigned to the variant due to the number of observational and intervention studies that have shown that controlling fat levels in the diet can be beneficial to these individuals. The carbohydrate gene score of 1 reflects the situation that observational and biochemical studies support the importance of carbohydrate for this variant, but the current levels of evidence from intervention studies are somewhat limited.

**Gene Score of 2:** The gene score of 2 is assigned to variants with reported associations with overweight or obese status in multiple publications, but with limited population sizes. Alternatively, associations with factors related to weight management may be reported, such as blood glucose levels or lipid levels, but less direct information on effects on BMI or body fat may be reported. General requirements for a gene score of 2 include: observational population based studies of 25-75 participants per study, or, alternatively, historical publications showing similar trends with populations of approximately 30 individuals per study, and also intervention studies with populations of less than 20 individuals showing an impact on weight related parameters. Conflicting reports in the literature may exist, but should be in the minority and ideally should be related to study design and/or population composition. Variants of eight genes-the ADRB2, ADRB3, APOA2, LIPC, IL6, FTO, IRS1, and TNF genes-have been given gene scores of 2.

The ADRB2 gene may affect the exercise category parameters. The Arg16Gly variant of the ADRB2 gene, similar to the Gln27Glu variant, has been associated with weight management. Several studies have shown a consistent trend of a positive impact of vigorous physical activity on fat mass and waist circumference in carriers of the Arg variant, leading to the assignment of a gene score of 2. In contrast, studies to date examining dietary factors have given an almost equal mix of positive and negative findings, so at the time of this writing, a gene score of 0 has been assigned to the Fat and Carbohydrate components.

The ADRB3 gene may also affect the exercise category parameters. Similar to the ADRB2 Arg16Gly variant, the ADRB3 Trp64Arg variant has been associated with weight management parameters and with vigorous physical activity showing an important role in reducing body fat, resulting in a gene score of 2 for Exercise. The dietary studies, however, have demonstrated conflicting results, leading to the assignment of a score of 0 for Fat and Carbohydrate components.

The APOA2 gene may affect the fat category parameters. The T>C variation has been associated with lower levels of the apoA-II protein, an important component of HDL particles. Several studies have shown an association of the C variant with elevated BMI, and individuals with the CC genotype have shown a positive response to weight management by limiting dietary saturated fat, resulting in a gene score assignment of 2 for fat. There is limited evidence on the role of carbohydrates, and only a single published study on the role of physical activity for carriers of this allele, so the carbohydrate and exercise gene scores are 0 for the C allele.

The LIPC gene may affect the fat category parameters. The -514C>T variant of the hepatic lipase gene has been associated with reduced activity, and associated with elevated BMI and visceral adiposity. Several studies have shown a positive impact of dietary saturated fat levels on HDL levels. However, some studies have reported no impact, leading to a gene score of 2 for fat. There is limited evidence for the role of carbohydrates and exercise at this time for this variant, so a gene score of 0 has been assigned for these parameters.

The IL6 gene may affect the fat category parameters. The -175G>C variant has been associated with decreased levels of the inflammatory cytokine IL-6 in plasma. The G (nonvariant) allele has been associated with greater difficulty in losing weight, and G carriers have a higher tendency to regain weight. Restricting saturated fat levels and raising polyunsaturated fat levels appear to be effective with this population, leading to the assignment of a gene score of 2 for fat. To date, there is limited evidence on the role of carbohydrates and physical activity for this gene, so a gene score of 0 has been assigned for these parameters.

The TNF gene may affect the fat category parameters. TNFa is an inflammatory cytokine, and the -308G>A promoter variant, which leads to higher expression of the cytokine, has been studied for many years for a potential role in weight management. A meta-analysis spanning several years has calculated an odds ratio of 1.23 for excess weight for carriers of the A allele. Studies have shown that dietary fat levels have an impact on weight loss in A allele carriers, leading to the assignment of a gene score of 2 for fat. To date, there is limited evidence on the role of carbohydrates and physical activity for this variant, so a gene score of 0 has been assigned for these parameters.

**Gene Score of 1:** The gene score of 1 is assigned to variants with reported associations with factors related to weight status, such as blood glucose levels or lipid levels, in smaller populations. Alternatively, associations with factors related to weight management may be reported. General requirements for a gene score of 1 include: observational population based studies of 10-25 participants per study, or, alternatively, historical publications showing similar trends with populations of a minimum 30 individuals per study, and intervention studies with 10 or more individuals showing an impact on weight related parameters, or a single long term intervention study (longer than 1 year in duration) with a population of greater than 200 individuals may be considered for assigning the gene score of 1. Conflicting reports in the literature may exist, but should be in the minority and ideally should be related to study design and/or population composition. Two genes--the LIPC and FABP2 gene-has been given a gene score of 1.

The algorithm disclosed herein may recommend one of the four diets to the customer as a genotype-appropriate diet based on the customer's cumulative score for the diet-related gene variants. Similarly, the disclosed algorithm may recommend either the moderate or vigorous exercise approach based on the customer's cumulative score for the exercise-related gene variants.

As an example, one algorithm that can be used entails the summing of the C scores and summing the F scores for each SNP in Table 2 and comparing these sums to thresholds. Exceeding the C threshold (>5 based on the arbitrary values assigned in Table 2) results in a carbohydrate-controlled diet, exceeding the F (>18) threshold results in a fat controlled diet, and exceeding both thresholds results in a fat-and-carbohydrate-controlled diet. If neither threshold is exceeded, then a balanced diet plan is indicated. Similarly, summing the E scores and exceeding the E (>10) threshold means that an intense level exercise plan is recommended; otherwise a moderate exercise level will suffice.

---

Calculation of Gene Score Sums

C Sum = SUM(SNP 01 C Score through SNP 13 C Score)

F Sum = SUM(SNP 01 F Score through SNP 13 F Score)

E Sum = SUM(SNP 01 E Score through SNP 13 E Score)

---

Comparison of Gene Score Sums to Thresholds

Dietary:

If C Sum $\geq$ 5 and F Sum < 18 then Diet Type = Carbohydrate Controlled Diet

If C Sum $\geq$ 5 and F Sum $\geq$ 18 then Diet Type = Fat and Carbohydrate Controlled Diet

If C Sum < 5 and F Sum < 18 then Diet Type = Balanced Diet

If C Sum < 5 and F Sum $\geq$ 18 then Diet Type = Fat Controlled Diet

---

Exercise:

If E Sum <10 then Exercise Intensity = Moderate (Level A)

If E Sum $\geq$ 10 then Exercise Intensity = Intense (Level B)

---

Diet Program:

P (OCC): Optimized Carbohydrate Control: P (OFCC). Optimized Fat and Carbohydrate Control

(continued)

Diet Program:

P (OBL): Optimized Balanced: (OFC): P (OFC): Optimized Fat Control

Exercise Intensity:

E (A): Moderate Exercise, level A: E (B) Intense Exercise. level B

$$f(C) = \sum_{S=1}^{S=13} S1\,(0-5) + S2\,(0-5) + S3(0-5) + \cdots$$

$$f(F) = \sum_{S=1}^{S=13} S1\,(0-5) + S2\,(0-5) + S3(0-5) + 3$$

$$f(E) = \sum_{S=1}^{S=13} S1\,(0-5) + S2\,(0-5) + S3(0-5) + \cdots$$

P(OCC), whereas - $f(C) \geq 5$:$f(F) < 18$

*P(OFCC), whereas ... $f(C) \geq 5$; $f(F) \geq 18$*

*P(OBL), whereas ... $f(C) < 5$; $f(F) < 18$*

*P(OFC), whereas ... $f(C) < 5$; $f(F) \geq 18$*

*E(A), whereas ... $f(E) < 10$*

*E(B), whereas ... $f(E) \geq 10$*

Of course, details of the algorithm and mathematical values assigned herein may vary for certain populations or as increased data become available as we continue to study these populations and the effect of various interventions on weight gain and health. For example, KLF14 (KRUPPEL-LIKE FACTOR 14; KLF14, aka BASIC TRANSCRIPTION ELEMENT-BINDING PROTEIN 5; BTEB5) may be a "master regulator," controlling the effect of a number of other genes that are linked to obesity, cholesterol and diabetes. Thus, rs4731702 (C>T) may be added to the panel when sufficient statistical information about this SNP become available, and adding another SNP will change the calculation given above accordingly. Further, other ways of mathematically obtaining an equivalent score may be devised, based on the general principles taught herein. However, the general methodology is broadly applicable even if assigned values and mathematical details may vary substantially.

From the evaluation of the data for the selected gene variants, the disclosed genetic diet algorithm describes four diets that may cover the variety of effects noted concerning diet-related weight management. One of four diets may be assigned based on normalized values, which include: (1) fat-controlled; (2) carbohydrate-controlled; (3) fat-and-carbohydrate-controlled; and (4) balanced diets.

Each diet may be custom-developed to the macronutrient specifications and numerous nutrition standards characteristic of healthy diets. For example, the fiber content in each diet may be at least 25g/day. The composition of the carbohydrates in some diets may be designed to deliver diets with a low glycemic load. Proteins that are lean in fat content and a mix of animal and plant proteins may be included. The diet can be developed to exclude trans fats and to emphasize monounsaturated fats relative to other fats. Additionally, in each diet, minimizing omega-6 polyunsaturated fats and including omega-3 polyunsaturated fats may be emphasized. In addition, each diet is designed to meet the nutritional requirements for adults according to the current U.S. Dietary Reference Intake guidelines.

The disclosed calorie levels for each diet may be appropriate for weight loss for most men and women. The recommended calorie levels may be based on the customer's BMI at the time the program is initiated. Calorie level is determined by a modified BMI/age based equation which utilizes height, weight and age. In one preferred embodiment:

Calculation of calorie needs:

If Gender = "Male" Then

CalorieNeeds = [(9.99 * (Weight * 0.45359237)) + (6.25 * (Height * 2.54)) - (CurrentAge * 4.92) + 5] - 500

(continued)

| |
|---|
| Calculation of calorie needs:<br>If Gender = "Female" Then<br>CalorieNeeds = [(9.99 * (Weight * 0.45359237)) + (6.25 * (Height * 2.54)) - (CurrentAge * 4.92) -161] - 500<br><br>If CalorieNeeds < 1400 Then CalorieCount = 1300<br>If CalorieNeeds >= 1400 And CalorieNeeds < 1800 Then CalorieCount = 1600<br>If CalorieNeeds >= 1800 Then CalorieCount = 1900 |

The target macronutrient composition for each diet is listed below in TABLE 3:

| Table 3: Diets | | | | |
|---|---|---|---|---|
| As Per Cent of Total Calories: | Fat-Controlled | Carbohydrate-Controlled | Fat & Carb-Controlled | Balanced |
| Carbohydrate | 55 | 40 | 45 | 55 |
| Protein | 25 | 30 | 35 | 20 |
| Fat | 20 | 30 | 20 | 25 |
| Calorie levels available: | 1300, 1600, 1900 | | | |

From the data evaluation relating to the gene variants' effect on exercise-related weight management, two exercise levels appear to be appropriate: (1) a moderate level of physical activity; or (2) a vigorous level of physical activity. The activity level recommendations may be given in metabolic equivalents ("METs"), a common measure of physical activity that allows the individual flexibility in choosing among a variety of types of physical activity.

All of the above SNPs are referenced in the SNP database at the National Center for Biotechnology Information, and are searchable online at ncbi.nlm.nih.gov/sites/entrez?db=snp. Population data and sequence information are also available in this database. Other sites, such as snpedia.com and snp.ims.u-tokyo.ac.jp/, are also available online.

Complete genomic sequences of the regions surrounding each SNP are likewise available in various databases, and thus the SNP can be tested for by any means known in the art. In a preferred method, the SNPs are profiled by designing primers based on the known sequences on either side of the SNP, PCR amplification of the region in question, and testing for a particular SNP by allele specific hybridization or sequence analysis, or in some instances by restriction enzyme analysis (e.g., where the SNP affects a restriction site) or protein analysis (e.g., where the SNP changes the sequence of a protein). Where a gene is maternally imprinted, any test for methylation status can be employed, or alternatively, both parents can be tested for allele status.

Furthermore, each of the above identified SNPS are known to be co-inherited (linked) with a number of other SNPs, and thus, detection of other alleles in the haplotype can easily substitute for a particular SNP recited herein. The international HapMap Project provides haplotype information at http://hapmap.ncbi.nlm.nih.gov/. Thus, when discussing testing for a particular SNP herein, it is understood that any SNP in linkage with the recited SNP is to be considered equivalent and exchangeable for the recited SNP.

For example, haplotype searches were performed using the HapMap browser, which shows results from the International HapMap Project: hapmap.org. The specific rs number of the SNPs of interest was entered into the search field, using the "HapMap Genome Browser Release #27 data source." SNPs with an r2 of at least 0.8 within a 40 kbp region were included and selected as sharing a haplotype of the SNPs identified herein, and the results are shown in Table 4. Haplotypes vary across populations and thus, the linked SNPs will vary according to which population is studied. However, Table 4 provides some exemplary linked SNPs. Additionally, some SNPs can be omitted or replaced with other more relevant SNPs without substantially changing the invention. However, in preferred embodiments at least 8/13, 9/13, or more preferably at last 10/13 or 11/13 SNPs are typed in the method. Most preferred is the full panel of 13 or more SNPs.

| Table 4: Linked SNPs | | | | | |
|---|---|---|---|---|---|
| Gene | rs number | Linked SNPs | Gene | rs number | Linked SNPs |
| ADRB2 | rs1042714 | None known yet | IL6 | rs1800795 | rs4722168 |
| ADRB2 | rs1042713 | None known yet | IL6 | rs1800795 | rs1829927 |

(continued)

| Table 4: Linked SNPs | | | | | |
|---|---|---|---|---|---|
| Gene | rs number | Linked SNPs | Gene | rs number | Linked SNPs |
| APOA2 | rs5082 | rs4073054 | IL6 | rs1800795 | rs6969258 |
| FABP2 | rs1799883 | rs10034661 | LIPC | rs1800588 | rs2070895 |
| FTO | rs9939609 | rs7202116 | LIPC | rs1800588 | rs8033940 |
| FTO | rs9939609 | rs7201850 | LIPC | rs1800588 | rs261334 |
| FTO | rs9939609 | rs7185735 | LIPC | rs1800588 | rs261332 |
| FTO | rs9939609 | rs9941349 | LIPC | rs1800588 | rs588136 |
| FTO | rs9939609 | rs9931494 | LIPC | rs1800588 | rs261342 |
| FTO | rs9939609 | rs17817964 | LIPC | rs2070895 | rs8033940 |
| FTO | rs9939609 | rs9930501 | LIPC | rs2070895 | rs261334 |
| FTO | rs9939609 | rs9930506 | LIPC | rs2070895 | rs261332 |
| FTO | rs9939609 | rs9932754 | LIPC | rs2070895 | rs588136 |
| FTO | rs9939609 | rs9922708 | LIPC | rs2070895 | rs261342 |
| FTO | rs9939609 | rs9922619 | LIPC | rs2070895 | |
| IL6 | rs1800795 | rs2069832 | PPARG | rs1801282 | rs1899951 |
| IL6 | rs1800795 | rs2069833 | PPARG | rs1801282 | rs4684848 |
| IL6 | rs1800795 | rs1474348 | PPARG | rs1801282 | rs2881654 |
| IL6 | rs1800795 | rs1474347 | TNFa | rs1800629 | None yet |
| IL6 | rs1800795 | rs1554606 | ACE | rs4343 | rs4344 |
| IL6 | rs1800795 | rs2069845 | ACE | rs4343 | rs4351 |
| IL6 | rs1800795 | rs12700390 | ACE | rs4343 | rs4353 |
| IL6 | rs1800795 | rs12700391 | ACE | rs4343 | rs4362 |
| IL6 | rs1800795 | rs7781534 | ADRB3 | rs4994 | None yet |

The disclosed embodiments are based on the belief that choosing dietary components and exercise approaches that complement the characteristics of the individual's unique gene variants may be helpful for individuals trying to manage their weight.

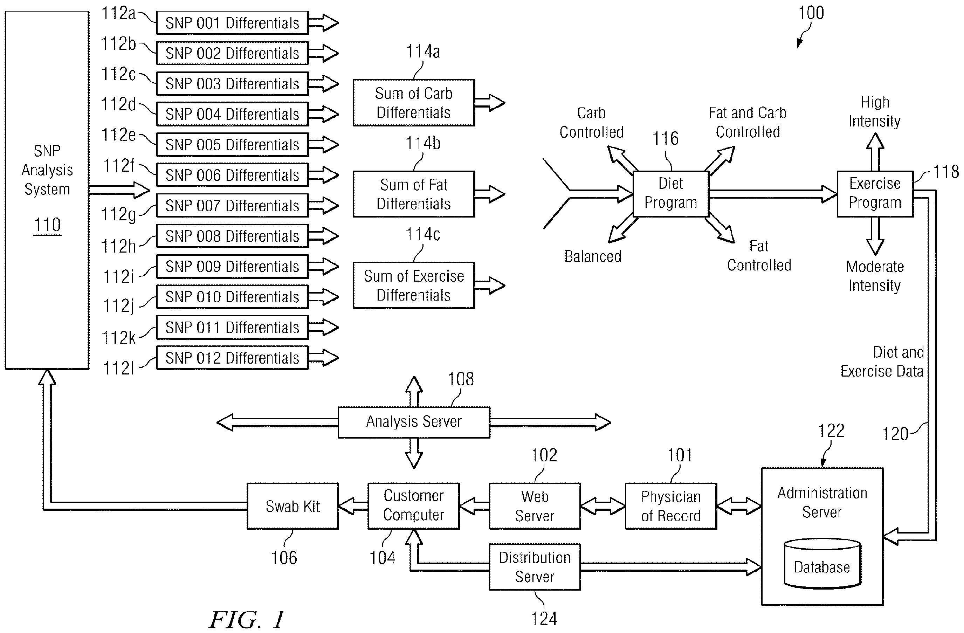

Referring now to FIGURE 1, a high level flow diagram of the genetic diet algorithm 100 is shown, in accordance with the present disclosure. The flow diagram 100 may begin at a website hosted by a web server 102 that a customer may log onto through his or her computer 104. The customer may enroll in a DNA testing program and may order a DNA test kit 106 in order to receive tailored diet and exercise programs. A physician of record 101 may approve the order of the DNA test kit 106 if required. The DNA test kit 106 may be shipped to the customer and the customer may provide a DNA sample (preferably from saliva) onto a swab in the DNA test kit 106. The DNA sample may be provided onto the swab from a cheek sample in an embodiment, although in other embodiments, the DNA sample may be provided from blood, urine, hair or other sample.

Once the customer has provided the DNA sample onto the DNA test kit 106, the customer may send the DNA test kit 106 to a SNP analysis system 110 for analysis. The SNP analysis system 110 may result, at a high level, with thirteen different single nucleotide polymorphism ("SNP") differentials 112 a through 1, each correlating to one of the 12 gene variants that have been identified to have a sufficiently strong effect on weight management, as discussed above and identified in TABLE 1. For example, in an embodiment, SNP differential 112a may correspond to the FTO gene in TABLE 1. The twelve different SNP differentials 112a-l may be unique to the customer based on the analysis of the DNA test kit 106 by the SNP analysis system 110. Based on the customer's unique SNP differentials 112a-l, sums of the differentials 114 may be separated to result in a sum of carb differentials 114a, a sum of fat differentials 114b, and a sum of exercise differentials 114c. The values for each SNP differential 112a-l may be found in TABLE 2, as discussed above.

The sum of these differentials 114a-c may be used to recommend a diet 116 to the customer as a genotype-appropriate diet based on the customer's cumulative score for the diet-related gene variants. The customer's unique SNP differentials 112 and the sums of the differentials 114a-c may then be used to recommend either a carb-controlled diet, a fat-controlled diet, a fat-and-carb-controlled diet, or a balanced diet 116. Although the diet 116 is broadly shown in FIG. 1 in these four categories, given the specifically measured SNP differentials 112a-l, a recommended diet can be specifically tuned for a particular customer. For example, particular types of foods avoiding long-chain dietary fatty acids can be recommended for customers whose SNP differentials 112 indicate the presence of the FABP2 gene, as shown above in TABLE 1. The present system and method provides for such indicated "tuning" in accordance with the measured SNP differentials 112.

Once a diet 116 is recommended, the customer or nutritional advisor may look to TABLE 3, as discussed above, to see their recommended daily calorie intake based on their gender and BMI, and then the percent of their total daily calories that should be carbohydrates, protein, and fat. For example, if a male with a BMI greater than 30 is recommended a fat-controlled diet based on his unique gene variants, the customer would be recommended a 1,900 daily calorie diet comprised of 55% carbohydrates, 25% proteins, and 20% fats, as well as any more specific guidance that is indicated.

Similarly, the disclosed algorithm may recommend either a moderate or vigorous exercise approach 118 based on the customer's cumulative score for the exercise-related gene variants.

Once the diet 116 and exercise 118 programs have been recommended based on the customer's SNP differentials 112a-l and their sum of differentials 114a-c, diet and exercise data 120 may be transferred to an administration server 122. The administration server 122 may comprise a client database that includes BMI data and gender data based on the diet and exercise data 120. After the diet and exercise data 120 are transferred to the administration server 122, the administration server 122 may transfer data to a distribution server 124 in order to process customer order fulfillment. The order fulfillment executed and instructed by the distribution server 124 may consist of shipping food, vitamins, supplements, etc. tailored to the customer's gene variants and diet and exercise programs. The order fulfillment may then be sent to the customer's desired shipping address.

An analysis server 108 may be working on nutritional genomics, diet formulation, and/or exercise planning and may take data from anywhere in the entire data flow 100 to conduct this research. These data may include a specific customer's SNP differentials 112a-l or their sum of differentials 114a-c, recommended diet 116 or exercise 118 programs, or the entire database of BMI and gender data in the administration server 122. Continuous modifications may be made to the system in the analysis server 108 based on customer feedback, and of course one or more servers may function in the various server roles.

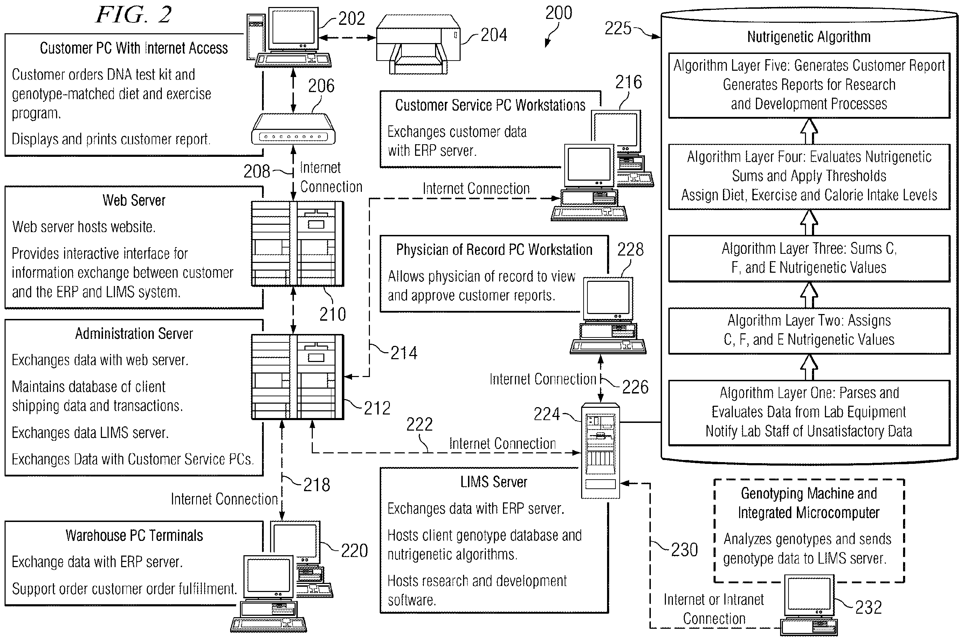

Referring now to FIGURE 2, a detailed system level diagram 200 of the flow diagram of FIG. 1 is shown, in accordance with the present disclosure. The system level diagram 200 may begin when the customer orders the DNA test, as discussed above in FIG. 1, on a customer personal computer ("PC") 202 connected to the internet, referring back to FIG. 2. The customer PC 202 may be connected to the internet via a modem 206, and the customer may order the DNA test kit from an online website. The customer may display and then print the customer report/order on a printer 204.

The modem 206 may connect the customer PC 202 to a web server 210 via an internet connection 208. The web server 210 may be configured to host the online website and provide an interactive interface for the exchange of information between the customer PC 202 and an administration server 212. The administration server 212 may be configured to exchange data with the web server 210. Additionally, the administration server 212 may be configured to maintain a database of client shipping data and transactions.

The administration server 212 may be configured to exchange data with one or more customer service PCs 216 via an internet connection 214. In addition, the administration server 212 may further be configured to exchange data with one or more warehouse PC terminals 220 via an internet connection 218. Furthermore, the administration server 212 may be configured to exchange data with a laboratory information management system ("LIMS") server 224 via an internet connection 222.

The LIMS server 224 may be connected to a genotyping machine and integrated microcomputer 232 via an internet or intranet connection 230. The LIMS server 224 operates code comprising instructions stored on a computer-readable medium. The genotyping machine and integrated microcomputer 232 may be configured to analyze genotypes and send genotype data to the LIMS server 224. The LIMS server 224 may then use this genotype data in the nutrigenetic algorithm 225.

The LIMS server 224 may also be configured to host a client genotype database and run one or more nutrigenetic algorithms 225. The LIMS server 224 operates code comprising computer instructions for executing the nutrigenetic algorithms 225 stored on a computer-readable medium.

The nutrigenetic algorithm 225, on a high level, may be broken down into five layers. In the first algorithm layer, the LIMS server 224 may parse and evaluate data from the lab results and may notify lab staff if the data is unsatisfactory. The presence of each of the 13 genes in the gene panel, disclosed above in TABLE 1, may be recorded. In the second algorithm layer, the LIMS server 224 may assign carbohydrate, fat, and exercise values to the data received from the lab results. The values assigned for carbohydrate, fat, and exercise correspond to the values disclosed above in TABLE 2. In the third algorithm layer, the LIMS server 224 may sum the carbohydrate, fat, and exercise values, as disclosed above in FIG. 1 at 114a-c. Referring back to FIG. 2, in the fourth algorithm layer, the LIMS server 224 may evaluate the nutrigenetic values, apply thresholds, and then assign diet, exercise, and calorie intake levels for the unique customer, as disclosed above in TABLE 3. In the fifth algorithm layer, the LIMS server 224 may generate a customer report, describing the recommended diet, exercise, and calorie intake levels based on the clients SNP differentials. In addition, the LIMS server 224 may generate reports for the research and development processes.

In addition to running the nutrigenetic algorithms 225, the LIMS server 224 may be configured to host research and development software. The LIMS server 224 may also be configured to be connected to a physician of record PC 228 via an internet connection 226. The physician of record PC 228 allows a physician of record to view and approve customer reports.

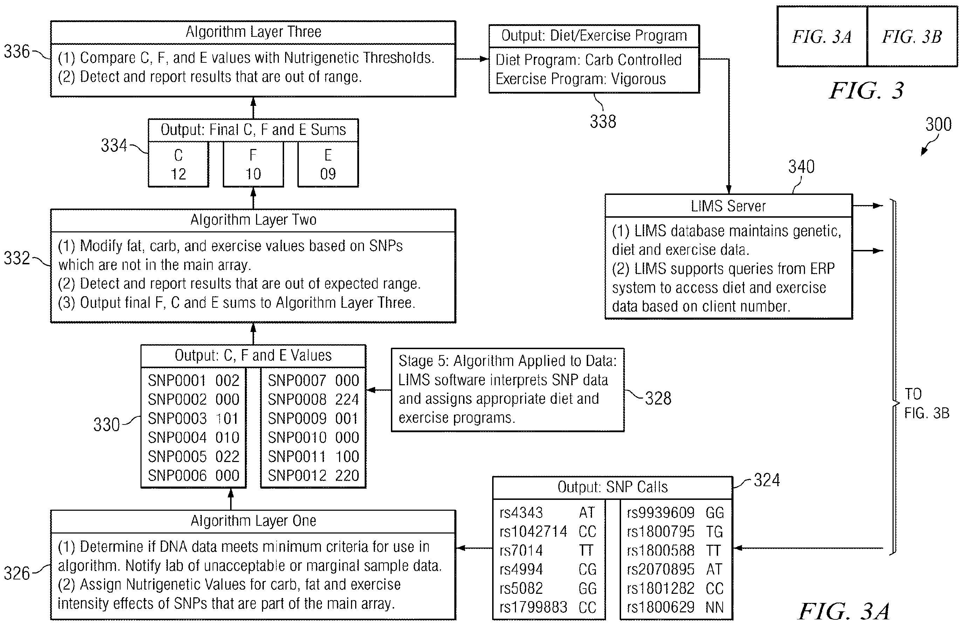

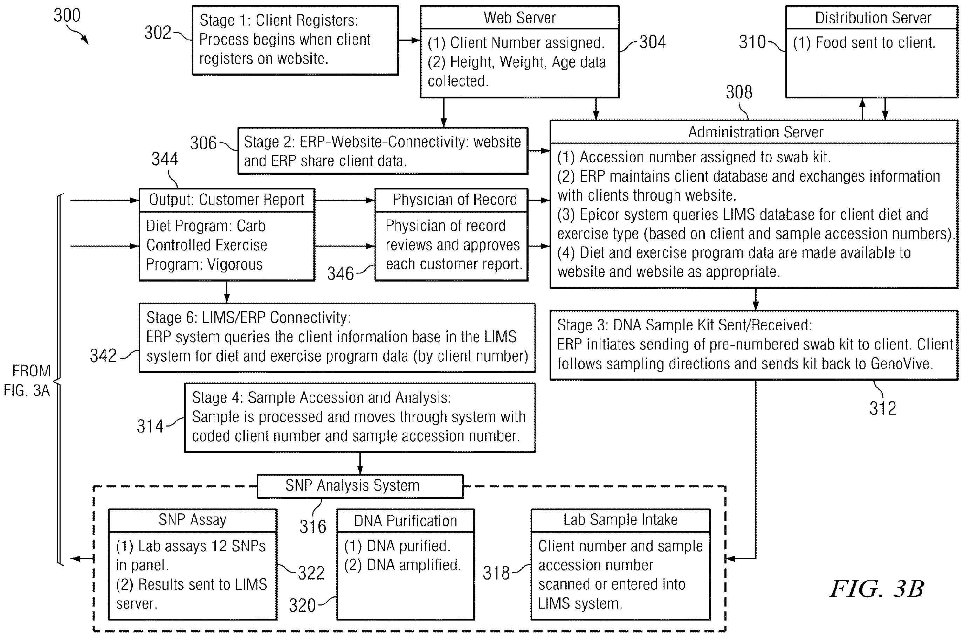

Referring now to FIGURE 3, a detailed flow diagram 300 combining the elements of FIGS. 1 and 2 is shown, in accordance with the present disclosure. The detailed flow diagram 300 may begin at stage one 302 where the customer registers on an interactive website hosted by a web server 304. The web server 304 may assign a client number to the customer, and the customer's height, weight, age, and gender data may be collected.

At stage two 306, the web server 304 and an administration server 308 may share the customer data from stage one 302. The administration server 308 may assign an accession number, which may be a unique identifier given to a DNA or protein sequence record to allow for tracking of different versions of that sequence record and the associated sequence over time, to a swab kit. The administration server 308 may also maintain a customer database and exchange information with customers through the website 304. In addition, the administration server 308 may query a LIMS server and database 340 for customer diet and exercise type based on customer and sample accession numbers. The administration server 308 may also make the diet and exercise program available to the website 304, as appropriate. The administration server 308 may be connected to a distribution server 310 that may fulfill orders and send them to the customer.

At stage three 312, a DNA sample kit may be sent and received, but the step order can of course be varied and this may occur at other times. The administration server 308 may initiate sending a pre-numbered swab kit to a customer. The customer is instructed to follow the DNA sampling instructions and then may send the pre-numbered swab kit back to the administration server 308 for processing.

At stage four 314, the DNA sample may be processed and moved through the administration server 308 with the coded sample accession number. A SNP analysis system 316 may take in DNA sample for processing at function 318. The customer number and sample accession number may be scanned or entered into the LIMS server and database 340. At function 320, the DNA sample may be purified and amplified. At function 322, the SNP analysis system 316 may assay the twelve SNP differentials featured in the nutrigenetic algorithm and send the results to a LIMS server and database 340. The output from the function 322 may be configured as SNP call outputs 324 and may include each of the twelve SNP differentials featured in the nutrigenetic algorithm, as disclosed above in TABLE 1.

In the first algorithm layer 326, as discussed above in FIG. 2, it must be determined if the sample DNA meets minimum criteria for use in the algorithm. If the sample data is unacceptable or marginal, the SNP analysis system 316 may be notified and the administration server 308 may ping the customer via the website hosted by the web server 304 and request an additional DNA sample. If the sample data is acceptable, nutrigenetic values may be assigned for carbohydrates, fat, and exercise intensity effects of the twelve SNP differentials that may be part of the main array.

At stage five 328, LIMS software may interpret the SNP data 324 and assign appropriate diet and exercise programs to the customer based on output values 330 for carbohydrate, fat, and exercise values. In FIG. 3, each SNP differential listed as an output value 330 may correspond to a gene in TABLE 1, where, in an embodiment, for example, SNP0001 may correspond to gene FTO. Each SNP differential listed as an output value 330 that corresponds to a specific gene may have a value for carbohydrates, fats, and exercise, as listed in TABLE 2. For example, in FIG. 3, the output values 330 for carbohydrate, fat, and exercise, respectively, may be 0, 0, and 2 for SNP0001; 0, 0, and 0 for SNP0002; 1, 0, and 1 for SNP0003; 0, 1, and 0 for SNP0004; 0, 2, and 2 for SNP0005; 0, 0, and 0 for SNP0006; 0, 0, and 0 for SNP0007; 2, 2, and 4 for SNP0008; 0, 0, and 1 for SNP0009; 0, 0, and 0 for SNP0010; 1, 0, and 0 for SNP0011; and finally 2, 2, and 0 for SNP0012.

In the second algorithm layer 332, the carbohydrate, fat, and exercise values may be modified based on any SNPs that may not be in the main array. In addition, any results that may be out of an expected range may be detected and reported. Finally, in the second algorithm layer 332, the final carbohydrate, fat, and exercise sums may be outputted as final output values 334.

At algorithm layer three 336, the final output values 334 may be compared with nutrigenetic thresholds. For example, in FIG. 3, the output value sums 334 may be 12 for carbohydrates, 10 for fats, and 9 for exercise, based on the SNP data output 324. In addition, any results that may be out of an expected range may be detected and reported. The algorithm layer three 336 may then output diet and exercise programs 338 tailored to the customer's carbohydrate, fat, and exercise values 334. For example, in FIG. 3, the recommended diet program could be a carb-controlled diet program and the recommended exercise program could be a vigorous exercise program.

The recommended diet and exercise programs 338 may then be sent to the LIMS server and database 340, which may be configured to maintain genetic, diet, and exercise data for each customer based on customer number. In addition, the LIMS server and database 340 may support queries from the administration server 308, allowing the administration server 308 to have access to diet and exercise data based on customer number.

At stage six 342, the administration server 308 may query by customer number the LIMS server and database 340 for diet and exercise program data for a unique customer. The LIMS server and database 340 may then output a customer report 344 and a physician of record report 346 and send the reports 344, 346 to the administration server 308. The customer report 344 may include the recommended diet and exercise programs for the customer queried by the administration server 308. For example, in FIG. 3, the recommended diet program may be a carb-controlled diet and the recommended exercise program may be a vigorous exercise program. The physician of record report 346 may be sent to the physician of record for the customer queried by the administration server 308, and may require the physician of record to review and approve each customer report 344.

In some embodiments, the method can also include supplying meals or components thereof that have been created to accord with the various diet plans. In preferred embodiments, the meals or components can also be divided into single serving portions that accord with the recommended caloric intake. Because food preferences are so variable, it may be preferable to provide meal components according to fat, protein, fiber, carbohydrate and caloric requirements that are then coded so that the client can pick ands choose the various components that combine to fit the recommended diet, and it will be easy to establish a computer based ordering system that automatically provides all options available to a client that meets their diet recommendations, but does not present choices that would be outside of the recommended plan. Alternatively, with a sufficiently efficient packaging facility, it will be possible to prepare complete single serving meals based on the selections made by the client. Thus, in this way, convenience and compliance are assured, while at the same time providing totally personal meals according to client's genetic profile and choices.

The method can also include providing exercise facilities, coaching and/or specific, detailed exercise programs. Further, the methods can be continuous and modified as a client's weight and lifestyle change.

## Claims

1. A method of weight management, comprising:

   a. measuring at least three of a client's values relating to weight, height, waist circumference, hip circumference, body mass index, waist-to-hip ratio, gender, and ethnicity,
   b. determining the allele of at least 10 non-repeating genetic variants in a biological sample obtained from said client, wherein said genetic variants are exercise or diet related variants,

      i. said exercise related variants are selected from a group consisting of rs9939609, rs4343, rs1042714, rs1042713, or rs4994, and
      ii. said diet related variants are selected from a group consisting of rs1042714, rs1799883, rs1800588, rs1800629, rs1800795, rs1801282, rs2070895, rs5082, rs9939609, or rs2943641,

   c. selecting a diet plan based on the results of a) and b)ii. and an exercise plan based on the results of a) and b)i., wherein said diet plan is carbohydrate controlled, fat controlled, fat and carbohydrate controlled or balanced diet, and said exercise plan is a moderate or intense plan

   wherein
   each of the determined genetic variants in step b) is assigned a Carbohydrate Score,
   Fat Score and Exercise Score based on the allele detected, and optionally also based on the values obtained in step a), summing said Carbohydrate Scores, wherein exceeding a first threshold results in a carbohydrate-controlled diet plan,
   summing wherein said Fat Scores, wherein exceeding a second threshold results in a fat controlled diet plan, wherein exceeding both first and second thresholds results in a fat-and-carbohydrate-controlled diet plan, but exceeding neither threshold results in a balanced diet plan, and
   summing said Exercise Scores, wherein exceeding a third threshold results in a vigorous exercise plan, and not exceeding said third threshold results in a moderate exercise plan.

2. The method of claim 1, wherein each of said client's weight, height, body mass index, waist-to-hip ratio, gender, and ethnicity are measured.

3. The method of claim 1, comprising determining the allele of at least 11 genetic variants.

4. The method of claim 1, comprising determining the allele of all 13 genetic variants.

5. The method of claim 2, comprising determining the allele of at least 11 genetic variants.

6. The method of claim 2, comprising determining the allele of all 13 genetic variants.

7. The method of claim 1, further comprising determining the allele of rs4731702.

8. A method of providing weight management services comprising:

    a. measuring at least three characteristics selected from weight, height, hip circumference, waist circumference, gender, and ethnicity from a client;
    b. determining the allele of at least 10 genetic variants known to be associated with weight gain from a biological sample obtained from said client and chosen from a group comprising rs1799883, rs1800588, rs1800629, rs1800795, rs1801282, rs2070895, rs4343, rs4994, rs5082, rs1042713, rs9939609, rs2943641, rs1042714;
    c. inputted the results of steps a) and b) into a computer algorithm, assigning each of the determined genetic variants in step b) a Carbohydrate Score, Fat Score and Exercise Score based on the allele detected, and optionally also based on the values measured in step a), and computing a score based on same, wherein said score is the summation of the values;
    d. outputting a weight management and exercise plan based on the scores obtained in step c);
    e. optionally providing prepared meals or components thereof to said client in accordance with said diet plan; and
    f. optionally providing detailed exercise plans and/or individual coaching in accordance with said exercise plan;
    and wherein summation of the values in step c) includes

summing said Carbohydrate Scores, wherein exceeding a first threshold results in a carbohydrate-controlled diet plan,
summing said Fat Scores, wherein exceeding a second threshold results in a fat controlled diet plan,
wherein exceeding both first and second thresholds results in a fat-and-carbohydrate-controlled diet plan, but exceeding neither threshold results in a balanced diet plan, and
summing said Exercise Scores, wherein exceeding a third threshold results in a vigorous exercise plan, and not exceeding said third threshold results in moderate exercise plan.

9. A method of providing weight management services, comprising:

    a. processing in a first processor a biological sample received from a client;
    b. receiving data representative of at least three characteristics selected from weight, height, hip circumference, waist circumference, gender, and ethnicity from said client;
    c. through the processing of the biological sample in the first processor, determining the allele of at least 10 genetic variants known to be associated with weight gain in said biological sample, wherein said genetic variants are exercise related variants or diet related variants,

        i. said exercise related variants are selected from a group consisting of rs9939609, rs4343, rs1042713 or rs4994, and rs1042714
        ii. said diet related variants are selected from a group consisting of rs1042714, rs1799883, rs1800588, rs1800629, rs1800795, rs1801282, rs2070895, rs5082, rs9939609, or rs2943641;

    d. computing in a second processor a score based upon the processing of the biological sample in step c) and the client data received in action b);
    e. determining in the second processor a weight management and exercise plan based on the score obtained from action d)

        i. wherein said weight management plan is a carbohydrate controlled, fat controlled, fat and carbohydrate controlled or balanced diet, and
        ii. wherein said exercise plan is a moderate or intense plan;

iii. wherein each of the determined genetic variants in step c) is assigned a Carbohydrate Score, Fat Score and Exercise Score based on the allele detected, and optionally also based on the values measured in step b),

iv. wherein said score computed in step d) involves

summing Carbohydrate Scores, wherein exceeding a first threshold results in a carbohydrate-controlled diet plan, summing Fat Scores, wherein exceeding a second threshold results in a fat controlled diet plan,
wherein exceeding both first and second thresholds results in a fat-and-carbohydrate-controlled diet plan, but exceeding neither threshold results in a balanced diet plan, and
summing Exercise Scores, wherein exceeding a third threshold results in a vigorous exercise plan, and not exceeding said third threshold results in moderate exercise plan;

f. transmitting the weight management and exercise plan to the client; and

g. optionally providing through a third processor for the selection and delivery of prepared meals or components thereof to the client in accordance with the diet plan of action f).

10. A method of weight management, comprising:

a. measuring at least three of a client's values relating to weight, height, waist circumference, hip circumference, body mass index, waist-to-hip ratio, gender, and ethnicity,

b. obtaining at least a partial sequence of nucleic acid from a biological sample obtained from said client;

c. analyzing said sequence and determining the allele of at least 10 non-repeating genetic variants in said client, wherein said genetic variants are exercise related variants or diet related variants,

i. said exercise related variants are selected from a group consisting of rs9939609, rs4343, rs1042714, rs1042713, or rs4994, and

ii. said diet related variants are selected from a group consisting of rs1042714, rs1799883, rs1800588, rs1800629, rs1800795, rs1801282, rs2070895, rs5082, rs9939609, or rs2943641,

d. selecting a diet plan based on the results of a) and c)ii., and an exercise plan based on the results of a) and c)i., wherein said diet plan is a carbohydrate controlled, fat controlled, fat and carbohydrate controlled or balanced diet, and said exercise plan is a moderate or intense plan whe
rein

each of the determined genetic variants in step c) is assigned a Carbohydrate Score, Fat Score and Exercise Score based on the allele detected, and optionally also based on the values obtained in step a),

summing said Carbohydrate Scores, wherein exceeding a first threshold results in a carbohydrate-controlled diet plan,

summing said Fat Scores, wherein exceeding a second threshold results in a fat controlled diet plan,

wherein exceeding both first and second thresholds results in a fat-and-carbohydrate-controlled diet plan,

wherein exceeding neither threshold results in a balanced diet plan, and

summing said Exercise Scores, wherein exceeding a third threshold results in a vigorous exercise plan,

wherein not exceeding said third threshold results in a moderate exercise plan, and

e. providing meals and exercise coaching to said client in accordance with said plans.

**Patentansprüche**

1. Verfahren der Gewichtskontrolle, umfassend:

a. Messung von wenigstens drei Werten eines Patienten bezüglich Gewicht, Körpergröße, Taillen- und Hüftumfang, BMI, Taille:Hüft-Verhältnis, Geschlecht und ethnischer Zugehörigkeit und

b. Ermittlung des Allels von wenigstens 10 sich nicht wiederholenden genetischen Varianten in einer biologischen Probe, erhalten von einem Patienten, bei dem die genetischen Varianten trainings- oder ernährungsbezogene Varianten darstellen,

i. die trainingsbezogenen Varianten ausgewählt werden aus einer Gruppe, bestehend aus rs9939609, rs4343, rs1042714, rs1042713 oder rs4994 und

ii. die ernährungsbezogenen Varianten ausgewählt werden aus einer Gruppe, bestehend aus rs1042714, rs1799883, rs1800588, rs1800629, rs1800795, rs1801282, rs2070895, rs5082, rs9939609 oder rs2943641

c. Auswahl eines Diätplans auf der Basis der Ergebnisse von a) und b) ii und eines Trainingsplans auf der Basis der Ergebnisse von a) und b) i., wobei der Diätplan eine kohlenhydrateingestellte, fetteingestellte, fett- und kohlenhydrateingestellte oder ausgeglichene Diät darstellt, und der Trainingsplan einen moderaten oder intensiven Plan darstellt, wobei

jeder der ermittelten genetischen Varianten auf Stufe b) ein Kohlenhydratwert, ein Fettwert und ein Trainingswert zugeordnet werden, basierend auf dem ermittelten Allel, und ggf. außerdem basierend auf den auf Stufe a) erhaltenen Werten,

Summierung der Kohlenhydratwerte unter Überschreitung erster Schwellenergebnisse in einem kohlenhydrateingestellten Diätplan,

Summierung der Fettwerte unter Überschreitung zweiter Schwellenergebnisse in einem fetteingestellten Diätplan, unter Überschreitung sowohl der ersten und zweiten Schwellenergebnisse in einem fett- und kohlenhydrateingestellten Diätplan, jedoch nicht unter Überschreitung keiner der beiden Schwellenergebnisse in einem ausgewogenen Diätplan, und

Summierung der Trainingswerte unter Überschreitung dritter Schwellenergebnisse in einem wirksamen Trainingsplan, jedoch ohne Überschreitung der dritten Schwellenergebnisse in einem moderaten Trainingsplan.

2. Verfahren nach Anspruch 1, wobei jeder der Werte bezüglich Gewicht, Körpergröße, Bodymassindex, Taille:Hüfte-Verhältnis, Geschlecht und ethnische Zugehörigkeit jedes Patienten gemessen wird.

3. Verfahren nach Anspruch 1, umfassend die Ermittlung des Allels von wenigstens 11 genetischen Varianten.

4. Verfahren nach Anspruch 1, umfassend die Ermittlung des Allels von sämtlichen 13 genetischen Varianten.

5. Verfahren nach Anspruch 2, umfassend die Ermittlung des Allels von wenigstens 11 genetischen Varianten.

6. Verfahren nach Anspruch 2, umfassend die Ermittlung des Allels von sämtlichen 13 genetischen Varianten.

7. Verfahren nach Anspruch 1, das außerdem noch die Ermittlung des Allels von rs4731702 umfasst.

8. Verfahren zur Gewährleistung der Gewichtskontrolle, umfassend:

a. Messung von wenigstens 3 Kennzeichen, ausgewählt unter Gewicht, Körpergröße, Hüftumfang, Taillenumfang, Geschlecht und ethnische Zugehörigkeit eines Patienten,
b. Ermittlung des Allels von wenigstens 10 genetischen Varianten, die dafür bekannt sind, dass sie im Zusammenhang stehen mit der Gewichtszunahme, aus einer biologischen Probe, erhalten von einem Patienten und ausgewählt aus der Gruppe, umfassend rs1799883, rs1800588, rs1800629, rs1800795, rs1801282, rs2070895, rs4343, rs4994, rs5082, rs1042713, rs9939609, rs2943641, rs1042714,
c. Input der Ergebnisse der Stufen a) und b) in einen Computeralgorithmus unter der Zuordnung jeder der ermittelten genetischen Varianten auf Stufe b) eines Kohlenhydratwerts, eines Fettwerts und eines Trainingswerts, basierend auf dem ermittelten Allel und ggf. auch basierend auf den auf Stufe a) gemessenen Werten und Berechnung eines darauf basierenden Werts, wobei dieser die Summe der Werte darstellt,
d. Output eines Gewichtskontroll- und Trainingsplans, basierend auf den auf Stufe c) erhaltenen Werten,
e. ggf. Versorgung des Patienten mit vorbereiteten Mahlzeiten oder deren Komponenten entsprechend dem Diätplan und
f. ggf. Bereitstellung detaillierter Trainingspläne und/oder eines patientenbezogenen Coachings entsprechend dem Trainingsplan, wobei die Summierung der Werte auf Stufe c) die Summierung der Kohlenhydratwerte unter Überschreitung erster Schwellenergebnisse in einem kohlenhydrateingestellten Diätplan,

die Summierung der Fettwerte unter Überschreitung zweiter Schwellenergebnisse in einem fetteingestellten Diätplan unter Überschreitung sowohl der ersten als auch der zweiten Schwellenergebnisse in einem fett- und kohlenhydrateingestellten Diätplan, jedoch nicht unter Überschreitung keiner der beiden Schwellenergebnisse in einem ausgewogenen Diätplan, und

die Summierung der Trainingswerte unter Überschreitung dritter Schwellenergebnisse in einem wirksamen Trainingsplan, jedoch nicht unter Überschreitung dritter Schwellenergebnisse in einem moderaten Trainingsplan.

9. Verfahren der Gewichtskontrolle, umfassend:

a. Bearbeitung einer von einem Patienten erhaltenen biologischen Probe in einem Prozessor,

b. Erhalt repräsentativer Daten für wenigstens 3 Kennzeichen, ausgewählt unter Gewicht, Körpergröße, Hüftumfang, Taillenumfang, Geschlecht und ethnische Zugehörigkeit des Patienten,

c. durch Verarbeitung der biologischen Probe in einem ersten Prozessor Ermittlung des Allels von wenigstens 10 genetischen Varianten, die dafür bekannt sind, dass sie mit der Gewichtszunahme in einer biologischen Probe verknüpft sind, wobei die genetischen Varianten trainings- oder diätbezogene Varianten darstellen,

> i. wobei die trainingsbezogenen Varianten ausgewählt werden aus einer Gruppe, bestehend aus rs9939609, rs4343, rs1042713, oder rs4994 und rs1042714
>
> ii. die diätbezogenen Varianten ausgewählt werden aus einer Gruppe, bestehend aus rs1042714, rs1799883, rs1800588, rs1800629, rs1800795, rs1801282, rs2070895, rs5082, rs9939609 oder rs2943641

d. Berechnung eines Werts, basierend auf der Verarbeitung der biologischen Probe auf Stufe c) und der auf Stufe b) erhaltenen Patientendaten in einem zweiten Prozessor und

e. Ermittlung eines Gewichtskontroll- und Trainingsplans mithilfe eines zweiten Prozessors basierend auf dem auf Stufe d) erzielten Wert,

> i. wobei der Gewichtskontrollplan eine kohlenhydrateingestellte, fetteingestellte, fett- und kohlenhydrateingestellte oder ausgewogene Diät ist, und
>
> ii. wobei der Trainingsplan ein moderater oder intensiver Plan ist,
>
> iii. wobei jede der auf Stufe c) ermittelten genetischen Varianten ein Kohlehydratwert, ein Fettwert und ein Trainingswert zugeordnet sind, basierend auf dem ermittelten Allel und ggf auf den auf Stufe b) gemessenen Werten und
>
> iv. wobei der auf Stufe d) errechnete Wert

die Summierung der Kohlenhydratwerte unter Überschreitung erster Schwellenergebnisse in einem kohlenhydrateingestellten Diätplan,

die Summierung der Fettwerte unter Überschreitung zweiter Schwellenergebnisse in einem fetteingestellten Diätplan

unter Überschreitung sowohl der ersten als auch der zweiten Schwellenergebnisse in einem fett- und kohlenhydrateingestellten Diätplan, jedoch nicht unter Überschreitung keiner der beiden Schwellenergebnisse in einem ausgewogenen Diätplan, und

Summierung der Trainingswerte unter Überschreitung dritter Schwellenergebnisse in einem wirksamen Trainingsplan, jedoch nicht unter Überschreitung dritter Schwellenergebnisse in einem moderaten Trainingsplan.

f. Übermittlung des Gewichtskontroll- und Trainingsplans an den Patienten und

g. ggf. Bereitstellung über einen dritten Prozessor zur Auswahl und Bereitstellung vorbereiteter Mahlzeiten oder Komponenten davon für den Patienten entsprechend dem Diätplan gemäß Stufe f).

**10.** Verfahren der Gewichtskontrolle, umfassend:

a. Messung von wenigstens drei gewichtsbezogenen Werten eines Patienten bezüglich Gewicht, Körpergröße, Taillen- und Hüftumfang, BMI, Taille:Hüft-Verhältnis, Geschlecht und ethnischer Zugehörigkeit

b. Gewinnung wenigstens einer Teilsequenz der Nukleinsäure aus einer aus einem Patienten erhaltenen biologischen Probe,

c. Analyse der Sequenz und Ermittlung des Allels von wenigstens 10 sich nicht wiederholenden genetischen Varianten des Patienten, wobei diese trainingsbezogene oder diätbezogene Varianten darstellen,

> i. die trainingsbezogenen Varianten ausgewählt werden aus einer Gruppe, bestehend aus rs9939609, rs4343, rs1042714, rs1042713, oder rs4994 und
>
> ii. die ernährungsbezogenen Varianten ausgewählt werden aus einer Gruppe, bestehend aus rs1042714, rs1799883, rs1800588, rs1800629, rs1800795, rs1801282, rs2070895, rs5082, rs9939609 oder rs2943641

d. Auswahl eines Diätplans auf der Basis der Ergebnisse von a) und b) ii und eines Trainingsplans auf der Basis der Ergebnisse von a) und b) i., wobei der Diätplan eine kohlenhydrateingestellte, fetteingestellte, fett- und kohlenhydrateingestellte oder ausgeglichene Diät darstellt, und der Trainingsplan einen moderaten oder intensiven Plan darstellt, wobei

jeder der ermittelten genetischen Varianten auf Stufe c) ein Kohlenhydratwert, ein Fettwert und ein Trainingswert zugeordnet werden, basierend auf dem ermittelten Allel, und ggf. außerdem basierend auf den auf Stufe a) erhaltenen Werten,
Summierung der Kohlenhydratwerte unter Überschreitung erster Schwellenergebnisse in einem kohlenhydrateingestellten Diätplan,
Summierung der Fettwerte unter Überschreitung zweiter Schwellenergebnisse in einem fetteingestellten Diätplan,
unter Überschreitung sowohl der ersten und zweiten Schwellenergebnisse in einem fett- und kohlenhydrateingestellten Diätplan, jedoch nicht unter Überschreitung keiner der beiden Schwellenergebnisse in einem ausgewogenen Diätplan, und
Summierung der Trainingswerte unter Überschreitung dritter Schwellenergebnisse in einem wirksamen Trainingsplan,
jedoch ohne Überschreitung der dritten Schwellenergebnisse in einem moderaten Trainingsplan, und
e. Bereitstellung von Mahlzeiten und Trainingscoaching für den Patienten entsprechend den genannten Plänen.

## Revendications

1. Procédé de gestion du poids, comprenant :

   a. la mesure d'au moins trois des valeurs d'un client relatives à son poids, sa taille, son tour de taille, son tour de hanche, son indice de masse corporelle, son rapport taille-hanche, son sexe et son groupe ethnique,
   b. la détermination de l'allèle d'au moins 10 variantes génétiques non répétitives dans un échantillon biologique obtenu dudit client, lesdites variantes génétiques étant des variantes liées à l'exercice ou au régime,

      i. lesdites variantes liées à l'exercice étant choisies dans un groupe constitué de rs9939609, rs4343, rs1042714, rs1042713 ou rs4994, et
      ii. lesdites variantes liées au régime étant choisies dans un groupe constitué de rs1042714, rs1799883, rs1800588, rs1800629, rs1800795, rs1801282, rs2070895, rs5082, rs9939609 ou rs2943641,

   c. le choix d'un régime alimentaire en fonction des résultats de a) et b)ii. et d'un programme d'exercice en fonction des résultats de a) et b)i., dans lequel ledit régime alimentaire est un régime à teneur réduite en glucides, à teneur réduite en matières grasses, à teneur réduite en matières grasses et en glucides ou équilibré, et ledit programme d'exercice est un programme modéré ou intensif

   dans lequel
   chacune des variantes génétiques déterminées à l'étape b) se voit attribuer un indice glycémique, un indice de gras et un niveau d'exercice en fonction de l'allèle détecté et, éventuellement, également en fonction des valeurs obtenues à l'étape a),
   l'addition desdits indices glycémiques, le dépassement d'un premier seuil entraînant un régime alimentaire à teneur réduite en glucides,
   l'addition desdits indices de gras, le dépassement d'un deuxième seuil entraînant un régime alimentaire à teneur réduite en matières grasses,
   dans lequel le dépassement à la fois du premier et du deuxième seuil entraîne un régime alimentaire à teneur réduite en matières grasses et en glucides, mais le non-dépassement de l'un ou l'autre des seuils entraîne un régime alimentaire équilibré, et
   l'addition desdits niveaux d'exercice, le dépassement d'un troisième seuil entraînant un programme d'exercice intense, et le non-dépassement dudit troisième seuil entraînant un programme d'exercice modéré.

2. Procédé selon la revendication 1, dans lequel on mesure chacun du poids, de la taille, de l'indice de masse corporelle, du rapport taille-hanche, du sexe et du groupe ethnique dudit client.

3. Procédé selon la revendication 1, comprenant la détermination de l'allèle d'au moins 11 variantes génétiques.

4. Procédé selon la revendication 1, comprenant la détermination de l'allèle des 13 variantes génétiques.

5. Procédé selon la revendication 2, comprenant la détermination de l'allèle d'au moins 11 variantes génétiques.

6. Procédé selon la revendication 1, comprenant la détermination de l'allèle des 13 variantes génétiques.

**7.** Procédé selon la revendication 1, comprenant en outre la détermination de l'allèle de rs4731702.

**8.** Procédé de fourniture de services de gestion du poids, comprenant :

a. la mesure d'au moins trois caractéristiques choisies parmi le poids, la taille, le tour de hanche, le tour de taille, le sexe et le groupe ethnique d'un client ;

b. la détermination de l'allèle d'au moins 10 variantes génétiques connues pour être associées à la prise de poids à partir d'un échantillon biologique obtenu dudit client et choisies dans un groupe comprenant rs1799883, rs1800588, rs1800629, rs1800795, rs1801282, rs2070895, rs4343, rs4994, rs5082, rs1042713, rs9939609, rs2943641, rs1042714 ;

c. une fois les résultats des étapes a) et b) entrés dans un algorithme informatique, l'attribution à chacune des variantes génétiques déterminées à l'étape b) d'un indice glycémique, d'un indice de gras et d'un niveau d'exercice en fonction de l'allèle détecté et, éventuellement, également en fonction des valeurs obtenues à l'étape a), et le calcul d'un score basé sur ceux-ci, ledit score étant la somme des valeurs ;

d. la production d'un programme d'exercice et de gestion du poids sur la base des scores obtenus à l'étape c) ;

e. éventuellement, la fourniture de repas préparés ou de leurs éléments constitutifs audit client conformément audit régime alimentaire ; et

f. éventuellement la fourniture de programmes d'exercices détaillés et/ou d'un accompagnement individuel conformément audit programme d'exercice ;

et dans lequel l'addition des valeurs de l'étape c) comprend

l'addition desdits indices de glycémie, le dépassement d'un premier seuil entraînant un régime alimentaire à teneur réduite glucides,

l'addition desdits indices de gras, le dépassement d'un deuxième seuil entraînant un régime alimentaire à teneur réduite en matières grasses,

dans lequel le dépassement à la fois du premier et du deuxième seuil entraîne un régime alimentaire à teneur réduite en matières grasses et en glucides, mais le non-dépassement de l'un ou l'autre des seuils entraîne un régime alimentaire équilibré, et

l'addition desdits niveaux d'exercice, le dépassement d'un troisième seuil entraînant un programme d'exercice intense, et le non-dépassement dudit troisième seuil entraînant un programme d'exercice modéré.

**9.** Procédé de fourniture de services de gestion du poids, comprenant :

a. le traitement dans un premier processeur d'un échantillon biologique reçu d'un client ;

b. la réception de données représentatives d'au moins trois caractéristiques choisies parmi le poids, la taille, le tour de hanche, le tour de taille, le sexe et le groupe ethnique dudit client ;

c. au travers du traitement de l'échantillon biologique dans le premier processeur, la détermination de l'allèle d'au moins 10 variantes génétiques connues pour être associées à la prise de poids dans ledit échantillon biologique, lesdites variantes génétiques étant des variantes liées à l'exercice ou des variantes liées au régime,

i. lesdites variantes liées à l'exercice étant choisies dans un groupe constitué de rs9939609, rs4343, rs1042713 ou rs4994, et rs1042714

ii. lesdites variantes liées au régime étant choisies dans un groupe constitué de rs1042714, rs1799883, rs1800588, rs1800629, rs1800795, rs1801282, rs2070895, rs5082, rs9939609 ou rs2943641 ;

d. le calcul dans un second processeur d'un score basé sur le traitement de l'échantillon biologique à l'étape c) et les données du client reçues à l'action b) ;

e. la détermination dans le second processeur d'un programme d'exercice et de gestion du poids en fonction du score obtenu de l'action d)

i. dans lequel ledit plan de gestion du poids est un régime à teneur réduite en glucides, à teneur réduite en matières grasses, à teneur réduite en matières grasses et en glucides ou un régime équilibré, et

ii. dans lequel ledit programme d'exercice est un programme modéré ou intensif ;

ii. dans lequel chacune des variantes génétiques déterminées à l'étape c) se voit attribuer un indice glycémique, un indice de gras et un niveau d'exercice en fonction de l'allèle détecté et, éventuellement, également en fonction des valeurs mesurées à l'étape b),

iv. dans lequel ledit score calculé à l'étape d) résulte de

l'addition des indices glycémiques, le dépassement d'un premier seuil entraînant un régime alimentaire à teneur réduite en glucides,
l'addition des indices de gras, le dépassement d'un deuxième seuil entraînant un régime alimentaire à teneur réduite en matières grasses,
dans lequel le dépassement à la fois du premier et du deuxième seuil entraîne un régime alimentaire à teneur réduite en matières grasses et en glucides, mais le non-dépassement de l'un ou l'autre des seuils entraîne un régime alimentaire équilibré, et
l'addition des niveaux d'exercice, le dépassement d'un troisième seuil entraînant un programme d'exercice intense, et le non-dépassement dudit troisième seuil entraînant un programme d'exercice modéré ;

f. la transmission du programme d'exercice et de gestion du poids au client ; et
g. éventuellement, la possibilité au travers d'un troisième processeur de choisir et de livrer des repas préparés ou leurs éléments constitutifs au client conformément au régime alimentaire de l'action f).

10. Procédé de gestion du poids, comprenant :

a. la mesure d'au moins trois des valeurs d'un client relatives à son poids, sa taille, son tour de taille, son tour de hanche, son indice de masse corporelle, son rapport taille-hanche, son sexe et son groupe ethnique,
b. l'obtention d'au moins une séquence partielle d'acide nucléique à partir d'un échantillon biologique obtenu dudit client ;
c. l'analyse de ladite séquence et la détermination de l'allèle d'au moins 10 variantes génétiques non répétitives chez ledit client, lesdites variantes génétiques étant des variantes liées à l'exercice ou au régime,

  i. lesdites variantes liées à l'exercice étant choisies dans un groupe constitué de rs9939609, rs4343, rs1042714, rs1042713 ou rs4994, et
  ii. lesdites variantes liées au régime étant choisies dans un groupe constitué de rs1042714, rs1799883, rs1800588, rs1800629, rs1800795, rs1801282, rs2070895, rs5082, rs9939609 ou rs2943641,

d. le choix d'un régime alimentaire en fonction des résultats de a) et c)ii et d'un programme d'exercice en fonction des résultats de a) et c)i., ledit régime alimentaire étant un régime à teneur réduite en glucides, à teneur réduite en matières grasses, à teneur réduite en matières grasses et en glucides ou équilibré, et ledit programme d'exercice étant un programme modéré ou intensif
dans lequel
chacune des variantes génétiques déterminées à l'étape c) se voit attribuer un indice glycémique, un indice de gras et un niveau d'exercice en fonction de l'allèle détecté et, éventuellement, également en fonction des valeurs obtenues à l'étape a),
l'addition desdits indices glycémiques, le dépassement d'un premier seuil entraînant un régime alimentaire à teneur réduite en glucides,
l'addition desdits indices de gras, le dépassement d'un deuxième seuil entraînant un régime alimentaire à teneur réduite en matières grasses,
dans lequel le dépassement à la fois du premier et du deuxième seuil entraîne un régime alimentaire à teneur réduite en matières grasses et en glucides,
dans lequel le non-dépassement de l'un ou l'autre des seuils entraîne un régime alimentaire équilibré, et
l'addition desdits niveaux d'exercice, le dépassement d'un troisième seuil entraînant un programme d'exercice intense,
le non-dépassement dudit troisième seuil entraînant un programme d'exercice modéré, et
e. la fourniture de repas et d'accompagnement pour l'exercice audit client conformément auxdits programme et régime.

*FIG. 1*

## FIG. 2

**Customer PC With Internet Access** — 202

Customer orders DNA test kit and genotype-matched diet and exercise program.

Displays and prints customer report.

204

206

208 — Internet Connection

200
225

**Nutrigenetic Algorithm**

Algorithm Layer Five: Generates Customer Report Generates Reports for Research and Development Processes

Algorithm Layer Four: Evaluates Nutrigenetic Sums and Apply Thresholds Assign Diet, Exercise and Calorie Intake Levels

Algorithm Layer Three: Sums C, F, and E Nutrigenetic Values

Algorithm Layer Two: Assigns C, F, and E Nutrigenetic Values

Algorithm Layer One: Parses and Evaluates Data from Lab Equipment Notify Lab Staff of Unsatisfactory Data

216

**Customer Service PC Workstations**

Exchanges customer data with ERP server.

Internet Connection

**Web Server**

Web server hosts website.

Provides interactive interface for information exchange between customer and the ERP and LIMS system.

210

228

**Physician of Record PC Workstation**

Allows physician of record to view and approve customer reports.

214

**Administration Server**

Exchanges data with web server.

Maintains database of client shipping data and transactions.

Exchanges data LIMS server.

Exchanges Data with Customer Service PCs.

212

Internet Connection — 226

Internet Connection

218 — Internet Connection

222

224

Internet Connection

**Warehouse PC Terminals**

Exchange data with ERP server.

Support order customer order fulfillment.

220

**LIMS Server**

Exchanges data with ERP server.

Hosts client genotype database and nutrigenetic algorithms.

Hosts research and development software.

230

**Genotyping Machine and Integrated Microcomputer**

Analyzes genotypes and sends genotype data to LIMS server.

Internet or Intranet Connection

232

EP 2 710 154 B1

**Algorithm Layer Three**

336 —
(1) Compare C, F, and E values with Nutrigenetic Thresholds.
(2) Detect and report results that are out of range.

**Output: Diet/Exercise Program**

Diet Program: Carb Controlled
Exercise Program: Vigorous

338

| FIG. 3A | FIG. 3B |
|---------|---------|

*FIG. 3*

300

340

**Output: Final C, F and E Sums**

334 —

| C | F | E |
|----|----|----|
| 12 | 10 | 09 |

**LIMS Server**

(1) LIMS database maintains genetic, diet and exercise data.
(2) LIMS supports queries from ERP system to access diet and exercise data based on client number.

**Algorithm Layer Two**

332 —
(1) Modify fat, carb, and exercise values based on SNPs which are not in the main array.
(2) Detect and report results that are out of expected range.
(3) Output final F, C and E sums to Algorithm Layer Three.

**Output: C, F and E Values**

330 —

| SNP0001 002 | SNP0007 000 |
|-------------|-------------|
| SNP0002 000 | SNP0008 224 |
| SNP0003 101 | SNP0009 001 |
| SNP0004 010 | SNP0010 000 |
| SNP0005 022 | SNP0011 100 |
| SNP0006 000 | SNP0012 220 |

Stage 5: Algorithm Applied to Data: LIMS software interprets SNP data and assigns appropriate diet and exercise programs.

—328

TO
FIG. 3B

**Algorithm Layer One**

326 —
(1) Determine if DNA data meets minimum criteria for use in algorithm. Notify lab of unacceptable or marginal sample data.
(2) Assign Nutrigenetic Values for carb, fat and exercise intensity effects of SNPs that are part of the main array.

**Output: SNP Calls**

324

| rs4343 | AT | rs9939609 | GG |
|--------|----|-----------|----|
| rs1042714 | CC | rs1800795 | TG |
| rs7014 | TT | rs1800588 | TT |
| rs4994 | CG | rs2070895 | AT |
| rs5082 | GG | rs1801282 | CC |
| rs1799883 | CC | rs1800629 | NN |

*FIG. 3A*

**300**

| Stage 1: Client Registers: Process begins when client registers on website. | → | **Web Server** |
|---|---|---|

302 — Stage 1: Client Registers: Process begins when client registers on website.

304 — **Web Server**
(1) Client Number assigned.
(2) Height, Weight, Age data collected.

310 — **Distribution Server**
(1) Food sent to client.

306 — Stage 2: ERP-Website-Connectivity: website and ERP share client data.

344

**Administration Server** 308
(1) Accession number assigned to swab kit.
(2) ERP maintains client database and exchanges information with clients through website.
(3) Epicor system queries LIMS database for client diet and exercise type (based on client and sample accession numbers).
(4) Diet and exercise program data are made available to website and website as appropriate.

**Output: Customer Report**
Diet Program: Carb Controlled Exercise Program: Vigorous

**Physician of Record**
Physician of record reviews and approves each customer report. 346

342 — Stage 6: LIMS/ERP Connectivity:
ERP system queries the client information base in the LIMS system for diet and exercise program data (by client number)

Stage 3: DNA Sample Kit Sent/Received:
ERP initiates sending of pre-numbered swab kit to client. Client follows sampling directions and sends kit back to GenoVive.

312

FROM FIG. 3A

314 — Stage 4: Sample Accession and Analysis:
Sample is processed and moves through system with coded client number and sample accession number.

**SNP Analysis System** 316

**SNP Assay**
(1) Lab assays 12 SNPs in panel.
(2) Results sent to LIMS server.
322

**DNA Purification**
(1) DNA purified.
(2) DNA amplified.
318
320

**Lab Sample Intake**
Client number and sample accession number scanned or entered into LIMS system.

*FIG. 3B*

EP 2 710 154 B1

28